# EUROPÄISCHE PATENTSCHRIFT

(11) **EP 3 322 983 B1**
(45) Veröffentlichungstag und Bekanntmachung des Hinweises auf die Patenterteilung: **11.03.2020**
(21) Anmeldenummer: 16736153.4
(22) Anmeldetag: 07.07.2016
(51) Int. Cl.: G01N 33/22, G01N 1/28, C10L 5/40

(54) **VERFAHREN ZUR AUFBEREITUNG VON ERSATZBRENNSTOFF SOWIE EIN VERFAHREN ZUR ANSCHLIESSENDEN ANALYSE DES ERSATZBRENNSTOFFS**
METHOD FOR PROCESSING SUBSTITUTE FUEL AND METHOD FOR SUBSEQUENTLY ANALYZING THE SUBSTITUTE FUEL
PROCÉDÉ POUR FOURNIR UN COMBUSTIBLE DE REMPLACEMENT ET PROCÉDÉ D'ANALYSE FINALE D'UN COMBUSTIBLE DE REMPLACEMENT

(30) Priorität: 15.07.2015 DE 102015111488
(43) Veröffentlichungstag der Anmeldung: 23.05.2018
(73) Patentinhaber: thyssenkrupp Industrial Solutions AG, 45143 Essen (DE); thyssenkrupp AG, 45143 Essen (DE)
(72) Erfinder: TEUTENBERG, Reinhard, 59423 Unna (DE); SCHNEBERGER, Jürgen, 59320 Ennigerloh (DE); BORNEFELD, Marc, 33617 Bielefeld (DE); MAIER, Oliver, 48167 Münster (DE); BENDIG, Uwe, 59069 Hamm (DE)
(74) Vertreter: thyssenkrupp Intellectual Property GmbH
(86) Internationale Anmeldenummer: PCT/EP2016/066066
(87) Internationale Veröffentlichungsnummer: WO 2017/009157

(56) Entgegenhaltungen:
- DE-A1- 19 837 177
- DE-A1-102007 006 137
- DE-A1-102010 031 528
- Anonymous: "Automatic milling and pressing system AMP", , Juni 2013 (2013-06), XP055301794, Germany Gefunden im Internet: URL:http://www.siebtechnik-gmbh.de/fileadm in/user_upload/PDF/en/Abt2/Wb239e.pdf [gefunden am 2016-09-12]

## Beschreibung

Die Erfindung betrifft ein Verfahren zur Aufbereitung von Ersatzbrennstoff sowie ein Verfahren zur anschließenden Analyse des Ersatzbrennstoffs.

In vielen Bereichen der Industrie, wie beispielsweise bei Zement-, Kalk- und Braunkohlekraftwerkten und großenteils auch in Industriekraftwerken, werden in zunehmendem Maße Ersatzbrennstoffe, insbesondere aus Hausmüll und Gewerbeabfällen, eingesetzt. Die Aufbereitungsintensität des Brennstoffs ist dabei von seinem Einsatzbereich sowie von unterschiedlichen Qualitätsanforderungen des Abnehmers, die eine eindeutige Spezifikation des abzunehmenden Ersatzbrennstoffs vorgeben, abhängig. Bedeutende Brennstoffparameter ergeben sich aus der Qualität der bei der Brennstoffherstellung genutzten Rohabfälle. Diese sind insbesondere Heizwert, Glührückstand (Veraschen) und Chlorgehalt. Je nach den emissionsrechtlichen Genehmigungen der Anlage, in welcher der Ersatzbrennstoff verwertet wird, werden Mindest- und Höchstwerte für Schwermetallgehalte definiert und verschiedene Forderungen an den Grad der Metallentfrachtung gestellt. An die Korngröße des Brennstoffs werden ebenfalls unterschiedliche Anforderungen gestellt. Besonders wichtig ist hier die Stückigkeit, also die Begrenzung der Stückgröße und Schüttdichte. Auch die Art der Lagerung und des Transportes sind von der Verwertungsweise des Brennstoffes abhängig.

Je höher die Qualitätsanforderungen durch die Abnehmer des Brennstoffs (Sekundärbrennstoff) sind, desto selektiver muss die eingesetzte Abfalltrennung vorgenommen werden. Schon bei der Eingangskontrolle muss eine Mindestqualität des Rohabfalls sichergestellt werden, um Verunreinigungen zu minimieren und eine hohe Brennstoffausbeute zu erhalten. Die Aufbereitung eines Abfallgemisches beginnt mit der Vorsortierung bzw. der Störstoffauslese, es folgen die Grobzerkleinerung und darauf die Siebklassierung sowie die Eisen-Magnetscheidung magnetisierbarer Metalle. Zur Abtrennung von hochkalorischen Fraktionen werden Windsichter eingesetzt. Dadurch werden Kunststofffolien und Papier angereichert bzw. flächige und flugfähige Bestandteile im Leichtstoffstrom gesammelt.

Um qualitätsgesicherte Ersatzbrennstoffe herzustellen, sind weitere Aufbereitungsschritte notwendig. Für den Einsatz in Zementwerken wird zum Beispiel eine Feinaufbereitung durchgeführt, wobei sensorische Sortierungen durch Nahinfrarotspektroskopie und Bilderkennungssysteme stattfinden. Es können auch weitere Metallsortierungen zur Anwendung kommen, sodass Nichteisenmetalle abgeschieden werden. Durch eine Sensorsortierung können weiterhin Chlorträger entfernt werden. Ein nachgeschalteter Windsichter kann dabei weitere Störstoffe aussortieren und durch Trocknungsschritte werden Lagerstabilität und Heizwert erhöht.

Anlagen, welche Ersatz- bzw. Sekundärbrennstoffe nutzen, müssen europaweit mindestens den Anforderungen der EU-Richtlinie 2000/76/EG zur Verbrennung und Mitverbrennung von Abfällen entsprechen. Im Deutschland gilt für die Abfallverbrennung und Mitverbrennungsanlagen außerdem die 17.BlmSchV.

Mit Energieanteilen von ungefähr 15% und darüber eignen sich Rohabfälle wie Altreifen, Kunststoffe, Industrie- und Gewerbeabfälle sowie Tiermehl und Tierfette zur Ersatzbrennstoffaufbereitung für den Einsatz in der Zementindustrie. Mit geringeren Energieanteilen werden unter anderem auch Altöl, Lösungsmittel sowie Siedlungsabfälle für die Aufbereitung genutzt.

Die zur Verfügung stehenden Ersatzbrennstoffe sind jedoch in ihrer Materialqualität oft sehr inhomogen. Um die immer enger definierten Emissionsgrenzwerte einhalten zu können, ist es von besonderem Interesse den Gehalt an Störstoffen, wie Schwefel, Schwermetallen und Chlor, der zum Einsatz kommenden Ersatzbrennstoffe zu kennen. Aufgrund der Heterogenität der genutzten Ersatzbrennstoffe ist es aber nach wie vor schwierig, belastbare Aussagen über die Materialqualität zu gewinnen.

In der Praxis werden entsprechend aufbereitete Ersatzbrennstoffe mit bestimmter Qualität vertrieben. Zur Analyse der eingesetzten Brennstoffe kommen insbesondere eine Nahinfrarotspektroskopie (NIR) oder Röntgenfluoreszenz-Analysen (RFA) zur Anwendung. Dabei lassen sich beispielsweise die Materialzusammensetzung, der Feuchtigkeitsgehalt und die Schadstoffgehalte analysieren. Des Weiteren kommen auch nasschemische Analysen zur Anwendung, die jedoch aufgrund der stark schwankenden Zusammensetzung der Ersatzbrennstoffe sehr aufwendig sind.

Stark schwankende Zusammensetzungen der Ersatzbrennstoffe bedingen aber auch entsprechend stark variierende Emissionen. Es kommt hinzu, dass die Ersatzbrennstoffe oftmals aus einem Gemisch unterschiedlicherer Ersatzbrennstoffe bestehen und dadurch bedingt auch der Brennwert innerhalb einer Charge schwanken kann. Für viele Verbrennungsprozesse, wie beispielsweise bei der Zementklinkerherstellung, kommt es jedoch entscheidend auf eine möglichst gleichmäßige Wärmezufuhr an, um die gewünschte Qualität des Endprodukts (Zementklinker) gewährleisten zu können. Die Forderungen an einen möglichst homogenen Brennwert und die Einhaltung von Emissionsgrenzwerten stehen somit einer Erhöhung des Anteils an Ersatzbrennstoffen in einem Brennprozess entgegen.

Aus der DE 198 37 177 A1 ist ein Verfahren zu Herstellung eines Hartbrennstoffes bekannt, wobei Ersatzbrennstoffe zunächst zerkleinert und anschließend in eine Reaktionseinrichtung verbracht werden, wo den Abfallstoffen anhaftendes und/oder gebundenes Wasser entfernt wird. Weiterhin werden der Reaktionseinrichtung Zuschlagstoffe, wie Kohle, Kalk und Katalysator in Abhängigkeit vom Zustand der Zusammensetzung des Mülls zugegeben und mit den Ersatzbrennstoffen vermischt.

Die DE 10 2007 006 137 A1 beschreibt ein Verfahren, bei dem Ersatzbrennstoffe mit Mineralien, wie Ton, Zeolith und Kalk, versetzt werden.

In der Veröffentlichung "Automatic milling and pressing system AMP" der Firma Siebtechnik Gmbh, Juni 2013, XP055301794, Deutschland, wird eine Maschine zur automatischen Probenaufbereitung beschrieben, bei der einer Probe ein Mahlzuschlagsmittel zugeführt und die Probe mit dem Zuschlagstoff dann vermahlen und verpresst wird.

Der Erfindung liegt daher die Aufgabe zugrunde, ein Verfahren zur Aufbereitung von Ersatzbrennstoff anzugeben, dass eine schnelle effiziente Analyse des Ersatzbrennstoffs ermöglicht.

Erfindungsgemäß ist das Verfahren zur Aufbereitung von Ersatzbrennstoff für eine anschließende Analyse des Ersatzbrennstoffes dadurch gekennzeichnet, dass
a. der Ersatzbrennstoff in zerkleinerter und homogenisierter Form bereitgestellt wird,
b. der bereitgestellte Ersatzbrennstoff anschließend zusammen mit einem Mineral und/oder einem anorganischen Salz auf eine Größe kleiner 100 µm vermahlen wird und
c. schließlich aus dem vermahlenen Gemisch eine analysefertige Probe gefertigt wird.

Dieses Aufbereitungsverfahren stellt repräsentative Proben bereit, die reproduzierbare Analysen ermöglichen. Der Ersatzbrennstoff sollte vorzugsweise mit einer Größe von weniger als 10 mm verarbeitet werden. Liegt der Ersatzbrennstoff nicht mit der gewünschten Körnung vor, erfolgt im Verfahrensschritt a) eine Zerkleinerung und Homogenisierung in einer Mühle. Liegt der Ersatzbrennstoff beispielsweise in Form von Fluff (flugfähige Fraktion) vor, kann die erste Zerkleinerung und Homogenisierung beispielsweise mit einer Rotorschere erfolgen. Je nach Art und Eigenschaften des Ersatzbrennstoffes kann gemäß Verfahrensschritt a) auch eine Vorzerkleinerung und Homogenisierung in einer ersten Mühle und eine Endzerkleinerung und Homogenisierung in wenigstens einer zweiten Mühle stattfinden, wobei als erste Mühle beispielsweise eine Rotorschere zum Einsatz kommt und die Endzerkleinerung und Homogenisierung beispielsweise in einer Schneidmühle oder einer Wirbelstrommühle durchgeführt wird.

Gemäß dem Verfahrensschritt b) wird der so aufbereitete Ersatzbrennstoff zusammen mit einem Mineral und/oder einem anorganischen Salz vermahlen, das vorzugsweise eine Körnung von 0,1 mm bis 8 mm aufweist. Dabei dient das Mineral und/oder anorganische Salz als Zerkleinerungshilfe und/oder Mahlhilfe und/oder Presshilfe. Des Weiteren kann es auch als Bindungshilfe, Auslösungshilfe und/oder Trennhilfe dienen. Vorzugsweise handelt es sich bei dem anorganischen Salz um eine Verbindung, die keinen oder wenig Einfluss auf die nachfolgende Analysentechnik hat. Kommt beispielsweise eine Röntgenfluoreszenzanalyse zur Anwendung, bietet sich der Einsatz von Lithiumtetraborat als Aufbereitungshilfe an.

Es dient dazu, einzelne schwer zu mahlende Bestandteile des Probenmaterials zunächst soweit zu zerschneiden, dass diese besser mahlbar werden (z.B. Alufolie, Plastikmüll). Folien weichen beim Mahlprozess eher aus. Grobkörniges Lithiumtetraborat unterstützt hierbei das Pulverisieren.

Bei dem Mineral handelt es sich beispielsweise um Korund, Siliziumcarbide, Quarz (Quarzsand) und Glas. Das Mineral sollte zweckmäßigerweise eine Mohs-Härte von mindestens 5 aufweisen, um eine weitere, effiziente Zerkleinerung bzw. Vermahlung des Ersatzbrennstoffes zu gewährleisten.

Gemäß einer bevorzugten Ausgestaltung des Verfahrens wird der Ersatzbrennstoff im Verfahrensschritt b) in Form einer flugfähigen Fraktion zusammen mit dem Mineral und anorganischen Stoff vermahlen.

Das vermahlene Gemisch wird im Verfahrensschritt c) vorzugsweise durch einen Pressvorgang, in eine bestimmte Form, beispielsweise eine Tablette oder einen Fladen, gebracht. Eine definierte Form vereinfacht das Handling bei der anschließenden Analyse und stellt auch eine definierte Größe für reproduzierbare Analysen dar. Des Weiteren werden durch die definierte Probenoberfläche einer gepressten Probe Genauigkeit (engl. precision) und Richtigkeit (engl. accuracy) der nachfolgenden Analyse verbessert. Sind die Proben beispielsweise in Stahlringe gepresst können sie mit RFID Transponder oder einem Code zur Vermeidung von Verwechselungen sicherer archiviert werden.

Die Erfindung bezieht sich auch auf ein Verfahren zur Aufbereitung und Analyse von Ersatzbrennstoff gemäß dem oben beschriebenen Verfahren und einer anschließenden Analyse der so gewonnenen, analysefertigen Proben. Bei der Analyse kann es sich insbesondere um eine molekülspektroskopische Analyse handeln. Es kommen insbesondere eine Röntgenfluoreszenzanalyse, eine Terahertz-Spektroskopie, aber auch eine Infrarotspektroskopie, einer Ramanspektroskopie oder eine UV-VIS-Spektroskopie in Betracht. Neben der Analyse der im Verfahrensschritt c) hergestellten analysefertigen Proben ist es natürlich denkbar und zweckmäßig, dass schon während oder nach den einzelnen Verfahrensschritten a), b) und c) Untersuchungen, insbesondere mittels Terahertz-Spektroskopie, vorgenommen werden. Die Terahertz-Spektroskopie ermöglicht insbesondere die Bestimmung von Heizwert, Feuchte, Kohlenstoff- und Chlorgehalt.

Die Terahertz-Spektroskopie ist eine zuverlässige Methode zur berührungslosen und zerstörungsfreien Untersuchung von Materialien, die sich insbesondere für den hier interessierenden Ersatzbrennstoff eignet. Die bei der Terahertz-Spektroskopie verwendeten elektromagnetischen Wellen liegen im Frequenzbereich zwischen 100GHz und 10Thz. Viele Moleküle zeigen in diesem Spektralbereich charakteristische Signaturen in ihren Absorptionsspektren, die einen chemischen Fingerabdruck bilden. Darüber hinaus sind viele für sichtbares Licht oder Infrarot undurchdringliche Stoffe für Terahertz-Wellen transparent. Die Terahertz-(Zeitbereichs)spektroskopie beruht auf der Erzeugung breitbandiger elektromagnetischer Strahlung durch ultrakurze Femtosekunden-Laserpulse und auf dem Nachweis mit dem Pump-Probe-Prinzip. Die Vorteile dabei sind eine kohärente Detektion der Terahertz-Wellen und damit eine hochauflösende Amplituden- und Phasenaufnahme des elektrischen Terahertz-Feldes im Zeitbereich. Diese Messtechnik unterdrückt inkohärente Strahlung, d. h. es gibt keine Störungen durch Raumtemperatur und Umgebungslicht.

So lassen sich mit Hilfe der Terahertz-(Zeitbereichs)Spektroskopie chemische Substanzen nachweisen und identifizieren. Dank der hohen Selektivität werden reine Substanzen oder Substanzgemische spezifisch detektiert. Im Unterschied zur IR- und Ramanspektroskopie, die empfindlich für intramolekulare Schwingungs- und Rotationsbewegungen sind, gibt die Terahertz-Spektroskopie Aufschluss zu den intramolekularen Bewegungen. So lassen sich neben dem Nachweis von Makromolekülen auch Aussagen über den Aggregatzustand, polymorphe Strukturen sowie die Kristallinität der Substanzen treffen. Die Terahertz-Spektroskopie kann daher vorteilhafterweise zusätzlich oder auch als Ersatz zur Röntgenbeugung verwendet werden, da sie schneller ist, minimale Probenpräparation erfordert und prinzipiell zur Online-Kontrolle genutzt werden kann. Messungen sind sowohl in Transmission als auch in Reflexion möglich.

Gemäß einer weiteren Ausgestaltung der Erfindung werden mittels chemometrischer Methoden chemische Informationen aus den Daten extrahiert, die bei der Analyse der analysefertigen Proben ermittelt werden. Die dabei gewonnenen chemischen Informationen werden zweckmäßigerweise mit selbstlernenden Algorithmen in einer Datenbank zusammengefasst und klassifiziert. Zur Strukturierung der Daten bzw. Datensätze kann dabei insbesondere eine Clusteranalyse zur Anwendung kommen.

Unter Chemometrie versteht man die Anwendung mathematischer und statistischer Methoden, um zuverlässig Informationen aus experimentellen Messdaten zu extrahieren. Bei der Chemometrie werden als Grundlage für eine Automatisierung in einer ersten Phase des Trainings oder Lernphase meist bekannte Stoffe unter vielen verschiedenen Bedingungen wiederholt gemessen. Auf Basis dieser Daten werden anschließend Expertensysteme oder Datenbanken aufgebaut. In der zweiten Phase, der Testphase werden dann weitere Messungen durchgeführt und gegen die Datenbank getestet.

Da es unmöglich ist, Messungen unter allen dankbaren Bedingungen durchzuführen, sollte es das Ziel beim Aufbau der Datenbank sein, weitestgehend nur substanzspezifische Informationen aufzunehmen. Im Umkehrschluss bedeutet dies, dass der nicht-substanzspezifische Informationsanteil aus den gemessenen Spektren entfernt werden muss. Hierzu gehören unter anderen die Einflüsse von Wasserdampflinien und der Partikelstreuung. Der Einfluss der nichtsubstanzspezifischen Informationsanteile kann durch eine geschickte Abfolge von spektralen Filtern minimiert werden. Nachdem alle Messdaten die informationsschärfende Filterfolge durchlaufen haben, wird eine Eigenschaftsreduktion durchgeführt. Als exploratives Werkzeug kann zum Beispiel die Hauptkomponentenanalyse "Principal Component Analysis" (PCA) verwendet werden. Die PCA beschreibt die hochdimensionalen Merkmale in einem alternativen, orthogonalen Raum. Die erste Hauptachse liegt in Richtung der höchsten Varianz, die zweite Hauptachse liegt rechtwinklig dazu in Richtung der zweithöchsten Varianz und so weiter. Oft genügen schon wenige Hauptachsen um einen Großteil der Informationen zu charakterisieren. Die Anteile der höheren Hauptachsen bleiben anschließend unberücksichtigt. Die Darstellung der ursprünglichen Messungen in den PCA-transformierten Raum zeigt oft schon eine sichtbare Trennung der Daten. Im Idealfall bilden sich für jedes Substanz einzelne Cluster.

Bei der Hauptkomponentenanalyse werden die umfangreichen Datensätze strukturiert, vereinfacht und veranschaulicht, indem eine Vielzahl statistischer Variablen durch eine geringere Zahl möglichst aussagekräftiger Linearkombinationen (die Hauptkomponenten) genährt wird. Wendet man dieses Verfahren auf die hier interessierende Analyse der Ersatzbrennstoffe an, so können beispielsweise Beziehungen zwischen den PVC-Anteilen und dem Chlorgehalt oder dem PVC-Anteil und dem Heizwert oder dem Chlorgehalt und dem Heizwert und dergleichen erkennbar und erlernbar sein.

Weitere Ausgestaltungen der Erfindung werden anhand der nachfolgenden Beschreibung und Zeichnung näher erläutert.

In der Zeichnung zeigen
- Fig. 1: ein Blockschaltbild des erfindungsgemäßen Verfahrens zur Aufbereitung von Ersatzbrennstoff und
- Fig. 2: ein detaillierteres Blockschaltbild des Verfahrens zur Aufbereitung von Ersatzbrennstoff in Verbindung mit der anschließenden Analyse des Brennstoffs.

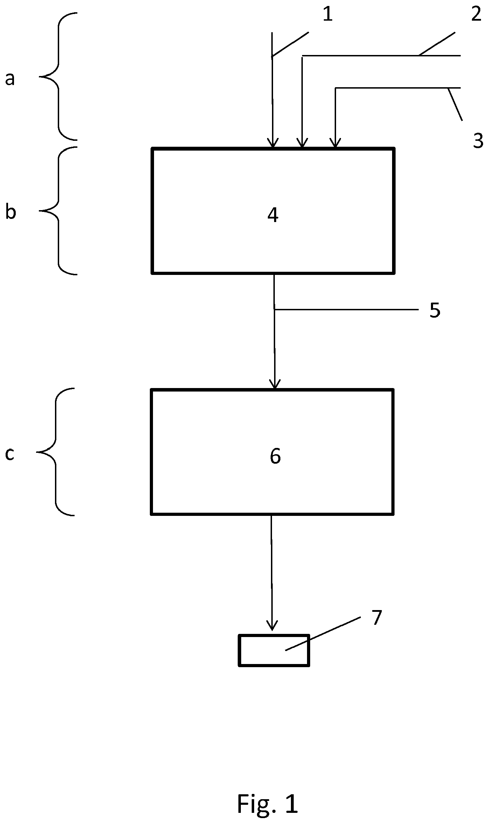

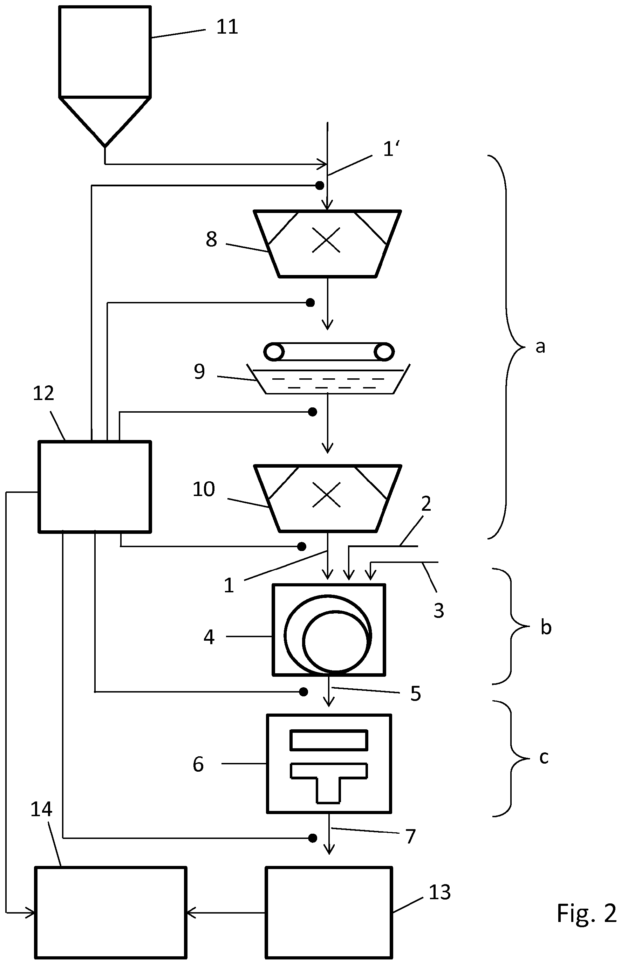

Gemäß dem in Fig. 1 gezeigten Blockschaltbildes erfolgt das Verfahren zur Aufbereitung von Ersatzbrennstoff für eine anschließende Analyse in drei Verfahrensschritten. Im ersten Verfahrensschritt a) wird ein Ersatzbrennstoff 1 in zerkleinerter und homogenisierter Form bereitgestellt. Diese Bereitstellung kann eine Zerkleinerung und Homogenisierung in ein oder mehreren Stufen umfassen, wie nachfolgend aus Fig. 2 ersichtlich wird. Der bereitgestellte Ersatzbrennstoff 1 sollte zweckmäßigerweise eine Größe von weniger als 10 mm aufweisen.

Im Verfahrensschritt b) werden der bereitgestellte Ersatzbrennstoff 1 anschließend zusammen mit einem Mineral 2, beispielsweise Quarz oder Korund und/oder einen anorganischen Salz 3, insbesondere Lithiumtetraborat, in einer Mühle 4 vermahlen. Bei der Mühle 4 handelt es sich beispielsweise um eine Scheibenschwingmühle, wobei das Gemisch aus bereitgestelltem Ersatzbrennstoff 1 und dem Mineral 2 und/oder dem anorganischen Salz 3 auf eine Größe < 100 µm vermahlen wird.

Im Verfahrensschritt c) wird schließlich aus dem vermahlenen Gemisch 5 eine analysefertige Probe 7 gefertigt. Dies kann insbesondere mit Hilfe einer Presse 6 erfolgen, die das vermahlene Gemisch 5 in einer bestimmten Form, beispielsweise einen Fladen oder eine Tablette, presst. Material wird z.B. in einen Stahlring gepresst, wobei der Stahlring an seiner Innenseite eine umlaufende Nut aufweisen kann, um ein besseres Anhaften des gepressten Probenmaterials zu gewährleisten.

Im Blockschaltbild gemäß Fig. 2 wird insbesondere der Verfahrensschritte a) in einer etwas detaillierteren Variante dargestellt und darüber hinaus mit einer anschließenden Analyse des aufbereiteten Ersatzbrennstoffs kombiniert.

Die Bereitstellung des Ersatzbrennstoffes 1 in zerkleinerter und homogenisierter Form gemäß dem Verfahrensschritt a) umfasst gemäß Fig. 2 eine Vorzerkleinerung und Homogenisierung in einer ersten Mühle 8, einen Magnetabscheider 9 und eine Endzerkleinerung und Homogenisierung in einer zweiten Mühle 10. Der noch zu zerkleinernde und homogenisierende Ersatzbrennstoff 1' wird beispielsweise von einem Lagerplatz oder einem Bunker 11 abgezogen, kann aber auch als Probe während der Zuführung des Ersatzbrennstoffes zu einer Verbrennungszone abgezweigt werden. Dieser Ersatzbrennstoff 1' wird zunächst für eine Vorzerkleinerung und Homogenisierung der ersten Mühle 8 zugeführt, die den Ersatzbrennstoff beispielsweise mittels Rotorscheren, einer Schneidmühle oder einer Wirbelstrommühle zerkleinert, anschließend gelangt der Ersatzbrennstoff in den Magnetabscheider 9, bevor er in der zweiten Mühle 10 einer Endzerkleinerung und Homogenisierung unterzogen wird. Die zweite Mühle kann ebenfalls durch eine Schneidmühle oder eine Wirbelstrommühle gebildet werden. Der Transport zwischen den Aggregaten erfolgt beispielsweise mittels Schwerkraft, über Schurren oder geeignete Transportmechanismen, wie Transportbänder, Kratzer oder durch Saugförderung, etc.

Der auf diese Weise bereitgestellte Ersatzbrennstoff 1 wird dann anschließend, wie schon oben beschrieben, gemäß dem Verfahrensschritten b) und c) weiter verarbeitet. An ausgewählten Messpositionen kann zudem vor und/oder nach jedem Zwischenschritt eine Untersuchung mittels Terahertz-Spektroskopie 12 erfolgen.

Die analysefertige Probe 7 wird anschließend in einer Analyseeinrichtung 13 einer Analyse unterzogen, wobei beispielsweise ein oder mehrere der nachfolgend aufgeführten Analyseverfahren zur Anwendung kommen können: Röntgenfluoreszenzanalyse, Terahertz-Spektroskopie, Elementaranalyse, Heizwertbestimmung, etc.

In einer Auswertungseinrichtung 14 werden die ermittelten Daten der Analyseeinrichtung 13 und der ggf. zum Einsatz kommenden Terahertz-Spektroskopie 12 weiter verarbeitet, wobei insbesondere chemometrische Methoden angewandt werden, um aus den Daten chemische Informationen zu extrahieren. Die gewonnenen chemischen Informationen können dann insbesondere mit selbstlernenden Algorithmen in ein oder mehreren Datenbanken zusammengefasst und klassifiziert werden, wobei zur Strukturierung der Daten bzw. Datensätze auch die Clusteranalyse zur Anwendung kommen kann.

## Patentansprüche

1. Verfahren zur Aufbereitung von Ersatzbrennstoff (1) für eine anschließende Analyse des Ersatzbrennstoffs (1), wobei
a. der Ersatzbrennstoff (1) in zerkleinerter und homogenisierter Form bereitgestellt wird,
b. der bereitgestellte Ersatzbrennstoff (1) anschließend zusammen mit einem Mineral (2) und/oder einem anorganischen Salz (3) auf eine Größe kleiner 100 µm vermahlen wird und
c. schließlich aus dem vermahlenen Gemisch (5) eine analysefertige Probe (7) gefertigt wird.

2. Verfahren nach Anspruch 1, **dadurch gekennzeichnet, dass** das Mineral (2) und/oder anorganische Salz (3) als Zerkleinerungshilfe und/oder Mahlhilfe und/oder Presshilfe wirken.

3. Verfahren nach Anspruch 1, **dadurch gekennzeichnet, dass** es sich bei dem anorganischen Salz (3) um ein Aufschlussmittel handelt.

4. Verfahren nach Anspruch 1, **dadurch gekennzeichnet, dass** das vermahlene Gemisch im Verfahrensschritt c) durch einen Pressvorgang in eine bestimmte Form gebracht wird.

5. Verfahren nach Anspruch 1, **dadurch gekennzeichnet, dass** der Ersatzbrennstoff (1) im Verfahrensschritt b) in Form einer flugfähigen Fraktion zusammen mit dem Mineral (2) und/oder dem anorganischen Salz (3) vermahlen wird.

6. Verfahren nach Anspruch 1, **dadurch gekennzeichnet, dass** der Ersatzbrennstoff (1) vor der Zugabe des Minerals (2) und/oder dem anorganischen Salzes (3) in einer Größe von weniger als 10 mm bereitgestellt wird.

7. Verfahren nach Anspruch 1, **dadurch gekennzeichnet, dass** das Mineral (2) und/oder das anorganische Salz (3) mit einer Körnung von 0,1 mm bis 8 mm mit dem Ersatzbrennstoff (1) im Verfahrensschritt b) vermahlen wird.

8. Verfahren nach Anspruch 1, **dadurch gekennzeichnet, dass** zumindest ein Teil der Zerkleinerung und der Homogenisierung gemäß Verfahrensschritt a) in einer Mühle (4) erfolgt.

9. Verfahren nach Anspruch 1, **dadurch gekennzeichnet, dass** gemäß Verfahrensschritt a) eine Vorzerkleinerung und Homogenisierung in einer ersten Mühle (8) und eine Endzerkleinerung und Homogenisierung in wenigstens einer zweiten Mühle (9) stattfinden.

10. Verfahren zur Aufbereitung und Analyse von Ersatzbrennstoff, **dadurch gekennzeichnet, dass** der Ersatzbrennstoff (1) zunächst entsprechend dem Verfahren gemäß Anspruch 1 aufbereitet wird, und anschließend die analysefertige Probe (7) einer Analyse unterzogen wird.

11. Verfahren nach Anspruch 10, **dadurch gekennzeichnet, dass** es sich bei der Analyse um eine molekülspektroskopische Analyse handelt.

12. Verfahren nach Anspruch 10, **dadurch gekennzeichnet, dass** mittels chemometrischer Methoden chemischen Informationen aus Daten extrahiert werden, die bei der Analyse der analysefertigen Probe ermittelt werden.

13. Verfahren nach Anspruch 12, **dadurch gekennzeichnet, dass** die mittels chemometrischer Methoden gewonnenen chemischen Informationen mit selbstlernenden Algorithmen in einer Datenbank zusammengefasst und klassifiziert werden.

14. Verfahren nach Anspruch 12 oder 13, **dadurch gekennzeichnet, dass** zur Strukturierung der Daten bzw. Datensätze eine Clusteranalyse angewendet wird.

15. Verfahren nach Anspruch 10, **dadurch gekennzeichnet, dass** das im Verfahrensschritt b) verwendete Mineral (2) und/oder anorganische Salz (3) so ausgewählt wird, dass es keinen störenden Einfluss auf die Analyse der analysefertigen Probe (7) hat.

## Claims

1. Process for treating substitute fuel (1) for subsequent analysis of the substituted fuel (1), wherein
a. the substitute fuel (1) is provided in comminuted and homogenized form,
b. the substitute fuel (1) provided is subsequently milled together with a mineral (2) and/or an inorganic salt (3) to a size of less than 100 µm and
c. a ready-to-analyse sample (7) is subsequently produced from the milled mixture (5).

2. Process according to Claim 1, **characterized in that** the mineral (2) and/or inorganic salt (3) act as comminution aid and/or milling aid and/or pressing aid.

3. Process according to Claim 1, **characterized in that** the inorganic salt (3) is a digestion agent.

4. Process according to Claim 1, **characterized in that** the milled mixture is brought to a particular shape by means of a pressing operation in process step c) .

5. Process according to Claim 1, **characterized in that** the substitute fuel (1) is milled in the form of a fraction entrainable in gas together with the mineral (2) and/or the inorganic salt (3) in process step b).

6. Process according to Claim 1, **characterized in that** the substitute fuel (1) is provided in a size of less than 10 mm before addition of the mineral (2) and/or the inorganic salt (3).

7. Process according to Claim 1, **characterized in that** the mineral (2) and/or the inorganic salt (3) is milled with a particle size of from 0.1 mm to 8 mm together with the substitute fuel (1) in process step b).

8. Process according to Claim 1, **characterized in that** at least part of the comminution and the homogenization according to process step a) is carried out in a mill (4).

9. Process according to Claim 1, **characterized in that** according to process step a), a preliminary comminution and homogenization takes place in a first mill (8) and a final comminution and homogenization takes place in at least one second mill (9) .

10. Process for treating and analysing substitute fuel, **characterized in that** the substitute fuel (1) is firstly treated by the process according to Claim 1 and the ready-to-analyse sample (7) is subsequently subjected to analysis.

11. Process according to Claim 10, **characterized in that** the analysis is a molecular spectroscopic analysis.

12. Process according to Claim 10, **characterized in that** chemical information is extracted by means of chemometric methods from data determined in the analysis of the ready-to-analyse sample.

13. Process according to Claim 12, **characterized in that** the chemical information obtained by means of chemometric methods are summarized and classified in a data bank using self-learning algorithms.

14. Process according to Claim 12 or 13, **characterized in that** a cluster analysis is employed for structuring the data or data sets.

15. Process according to Claim 10, **characterized in that** the mineral (2) and/or inorganic salt (3) used in process step b) is selected so that it does not have any interfering effect on the analysis of the ready-to-analyse sample (7).

## Revendications

1. Procédé de préparation d'un combustible de substitution (1) pour ensuite procéder à une analyse du combustible de substitution (1),
a. le combustible de substitution (1) étant mis à disposition sous une forme concassée et homogénéisée,
b. le combustible de substitution (1) mis à disposition étant ensuite moulu conjointement avec un minéral (2) et/ou un sel anorganique (3) à une taille inférieure à 100 µm et
c. un échantillon (7) prêt pour l'analyse étant ensuite produit à partir du mélange (5) moulu.

2. Procédé selon la revendication 1, **caractérisé en ce que** le minéral (2) et/ou le sel anorganique (3) agissent comme un auxiliaire de concassage et/ou un auxiliaire de mouture et/ou un auxiliaire de pressage.

3. Procédé selon la revendication 1, **caractérisé en ce que** le sel anorganique (3) est un agent de dissolution.

4. Procédé selon la revendication 1, **caractérisé en ce qu'**à l'étape c), le mélange moulu est amené dans une forme déterminée par une opération de pressage.

5. Procédé selon la revendication 1, **caractérisé en ce qu'**à l'étape b), le combustible de substitution (1) est moulu sous une forme dispersible conjointement avec un minéral (2) et/ou un sel anorganique (3).

6. Procédé selon la revendication 1, **caractérisé en ce qu'**avant l'apport du minéral (2) et/ou du sel anorganique (3), le combustible de substitution (1) est mis à disposition dans une taille inférieure à 10 mm.

7. Procédé selon la revendication 1, **caractérisé en ce qu'**à l'étape b), le minéral (2) et/ou le sel anorganique (3) sont moulus avec le combustible de substitution (1) à une granulométrie de 0,1 mm à 8 mm.

8. Procédé selon la revendication 1, **caractérisé en ce qu'**au moins une partie du concassage et de l'homogénéisation selon l'étape a) est réalisée dans un broyeur (4).

9. Procédé selon la revendication 1, **caractérisé en ce que** selon l'étape a), un pré-concassage et une homogénéisation ont lieu dans un premier broyeur (8) et un concassage final et une homogénéisation ont lieu dans au moins un deuxième broyeur (9).

10. Procédé de préparation et d'analyse d'un combustible de substitution, **caractérisé en ce que** le combustible de substitution (1) est tout d'abord préparé conformément à un procédé selon la revendication 1 et ensuite l'échantillon (7) prêt pour l'analyse est soumis à une analyse.

11. Procédé selon la revendication 10, **caractérisé en ce que** l'analyse est une analyse spectroscopique moléculaire.

12. Procédé selon la revendication 10, **caractérisé en ce que** des informations chimiques sont extraites au moyen de méthodes chimiométriques des données qui sont déterminées lors de l'analyse de l'échantillon prêt pour l'analyse.

13. Procédé selon la revendication 12, **caractérisé en ce que** les informations chimiques obtenues au moyen des méthodes chimiométriques sont rassemblées et classifiées dans une base de données avec des algorithmes d'autoapprentissage.

14. Procédé selon la revendication 12 ou 13, **caractérisé en ce qu'**une analyse de groupement est appliquée pour la structuration des données ou des jeux de données.

15. Procédé selon la revendication 10, **caractérisé en ce que** le minéral (2) et/ou le sel anorganique (3) utilisé à l'étape b) est sélectionné de telle sorte qu'il n'a aucune influence perturbatrice sur l'analyse de l'échantillon (7) prêt pour l'analyse.
